# FASCICULE DE BREVET EUROPEEN

(11) **EP 3 481 171 B1**
(45) Date de publication et mention de la délivrance du brevet: **07.04.2021**
(21) Numéro de dépôt: 17730434.2
(22) Date de dépôt: 08.06.2017
(51) Int. Cl.: A01D 44/00

(54) **PROCÉDÉ ET UNITÉ DE RECOLTE ET DE TRAITEMENT DES JACINTHES D'EAU**
VERFAHREN UND VORRICHTUNG ZUM ERNTEN VON HYAZINTHEN
METHOD AND DEVICE FOR HARVESTING HYACINTHS

(30) Priorité: 07.07.2016 FR 1656535
(43) Date de publication de la demande: 15.05.2019
(73) Titulaire: IN-Between SA, 1428 Braine l'Alleud (BE)
(72) Inventeur: BAHADORANI, Rebeka, 1428 Braine l'Alleud (BE); BONO, Pierre, 10300 Sainte Savine (FR)
(74) Mandataire: Cabinet Bleger-Rhein-Poupon
(86) Numéro de dépôt international: PCT/EP2017/063923
(87) Numéro de publication internationale: WO 2018/007090

(56) Documents cités:
- WO-A2-92/21227
- GB-A- 369 263
- US-A- 4 222 217
- US-A- 5 487 258

## Description

La présente invention entre dans le domaine des procédés de récolte et de traitement des jacinthes d'eau.

La jacinthe d'eau ou « *Eichhornia crassipes* » est une plante aquatique d'eau douce, de type macrophyte aquatique. Cette espèce de plante est très envahissante. Elle pose problème par sa croissance rapide et durant sa décomposition. En effet, sa croissance, rapide et étendue, impacte l'écosystème des eaux douces. La propagation de la jacinthe d'eau dans son environnement peut notamment étouffer les espèces natives, en formant, au niveau des strates inférieures, des tapis denses mono-spécifiques, les privant de lumière vitale.

De plus, lors de sa décomposition, la jacinthe d'eau libère, dans son environnement aquatique, des nutriments pouvant mener à l'eutrophisation ou à l'anoxie du milieu, ce qui représente un danger pour la biodiversité aquatique pouvant engendrer la mort de nombreuses espèces cohabitant avec les jacinthes d'eau.

En outre, les graines de jacinthes d'eau sont pérennes dans le temps, le temps de dormance des graines est de vingt ans. Ces graines supportent la sécheresse et germent dès leur immersion en milieu aquatique. Ainsi, même en cas de récolte, lors du stockage, il existe un risque de contamination, c'est à dire de germination des graines, en particulier si l'environnement de stockage est humide. En effet, pour éviter la propagation dans son milieu naturel des jacinthes d'eau, les documents US -A-5487258, WO -A-9221227, ou encore, GB -A-369263 divulguent des dispositifs de récolte et de traitement, notamment par broyage, des jacinthes d'eau. Néanmoins, dans ces dispositifs connus, le broyage de la jacinthe d'eau se fait directement après la récolte, en milieu humide. Ainsi, dans ces conditions humides, même après broyage, le risque de germination des graines de jacinthes d'eau est élevé dans la zone de stockage. Le traitement de la jacinthe d'eau après récolte est donc insatisfaisant car il présente un risque de germination des graines a postériori non désiré.

Ainsi, il est nécessaire de pouvoir éviter ce risque de germination des graines de jacinthes d'eau lors du stockage pour permettre leur valorisation dans le milieu industriel.

C'est pourquoi, pour éviter les phénomènes susmentionnés, il est nécessaire de trouver une solution facile et rapide pour récolter, traiter et valoriser industriellement la jacinthe d'eau.

La présente invention a pour but de pallier les inconvénients de l'état de la technique, en proposant un procédé de récolte et de traitement des jacinthes d'eau dans lequel on réalise les étapes suivantes :
a) on coupe les jacinthes d'eau présentes dans un milieu aquatique ;
b) on collecte les jacinthes d'eau à bord d'une barge à quai ;
c) on transfère les jacinthes d'eau collectées sur des moyens d'égouttage, de type égouttoirs, à proximité de la rive du quai ;
d) on lave les jacinthes égouttées avec de l'eau propre ;
e) on égoutte sur tapis vibrants les jacinthes d'eau lavées pour éliminer en partie le surplus d'eau ;
f) on sèche les jacinthes d'eau sur un séchoir à une température comprise entre 50 et 100°C pendant une durée comprise entre 5h et 17h ;
g) on stérilise à la vapeur à une température comprise entre 80 et 100°C, afin de stériliser les jacinthes d'eau et leurs graines ;
h) on broie les jacinthes d'eau et leurs graines stérilisées afin de les valoriser.

De plus, selon d'autres caractéristiques du procédé de l'invention :
- on récupère les eaux résiduelles issues de l'égouttage de l'étape c), on traite lesdites eaux résiduelles par stérilisation UV au sein d'une station d'épuration pour les rejeter dans le milieu aquatique d'origine des jacinthes d'eau ;
- l'eau propre de l'étape d) consiste en de l'eau prélevée à partir du milieu aquatique d'origine des jacinthes d'eau, ladite eau prélevée étant pompée à l'aide d'une station de pompage et filtrée au travers d'une grille filtrante comprenant un diamètre d'ouverture compris entre 6 et 10 mm de manière à la rendre propre pour l'étape de lavage ;
- on réalise l'étape d) en lavant au moins deux fois à l'eau propre les jacinthes égouttées dans des moyens de lavage, de type conteneur de lavage;
- après l'étape g) de stérilisation et avant l'étape h) de broyage, on sèche les jacinthes d'eau stérilisées par un système d'air chaud puisé ;
- après l'étape h) de broyage, on réalise une micronisation des broyats obtenus afin de permettre leur utilisation et leur valorisation dans le domaine de la plasturgie ou des matériaux d'isolation ;
- l'étape f) de séchage est réalisée à l'aide d'un séchoir vertical à air chaud ;
- après l'étape de séchage f) et avant l'étape g) de stérilisation, on concentre les jacinthes d'eau séchées dans des moyens de concentration, de type concentrateur ;
- après l'étape d) de lavage desdites jacinthes d'eau égouttées, on coupe ces dernières en au moins trois ou quatre tronçons.

La présente invention concerne également une unité de récolte et le traitement des jacinthes d'eau permettant la réalisation du procédé de l'invention et dans cet ordre :
- Une barge flottante à proximité de la rive, ladite barge étant équipée de moyens de coupe des jacinthes d'eau, de moyens de collecte et de stockage des jacinthes d'eau découpées, et de moyens de transfert desdites jacinthes d'eau vers la rive, lesdits moyens de transfert étant destinés à transférer lesdites jacinthes d'eau vers des moyens d'égouttage destinés à égoutter les jacinthes d'eau collectées ;
- Des moyens de déplacement des jacinthes d'eau égouttées vers des moyens de lavage connectés à des moyens d'égouttage supplémentaires ;
- Des moyens de séchage en sortie desdits moyens d'égouttage supplémentaires ;
- Des moyens de concentration des jacinthes d'eau séchées en sortie desdits moyens de séchage;
- Des moyens de stérilisation des jacinthes d'eau concentrées en sortie desdits moyens de concentration;
- Des moyens de broyage des jacinthes d'eau stérilisées en sortie desdits moyens de stérilisation.

D'autres caractéristiques et avantages de l'invention ressortiront de la description détaillée qui va suivre des modes de réalisation non limitatifs de l'invention, en référence aux figures annexées, dans lesquelles :
- la figure 1 représente schématiquement une vue de l'unité de récolte et de traitement des jacinthes d'eau ;
- la figure 2 représente schématiquement une vue du séchoir vertical à air chaud de l'unité de récolte et de traitement des jacinthes d'eau.

La présente invention concerne un procédé de récolte et de traitement des jacinthes d'eau.

La récolte des jacinthes d'eau se fait à l'aide d'une barge flottante 1 équipée de moyens de coupe, de moyens de collecte et de stockage 110 de jacinthes d'eau découpées.

Ainsi, la mise en œuvre du procédé de l'invention se fait en réalisant successivement les étapes suivantes :
a) On coupe les jacinthes d'eau présentes dans un milieu aquatique ;
b) On collecte les jacinthes d'eau à bord d'une barge 1 à quai.

Une fois récoltées, le procédé de l'invention permet de transférer les jacinthes d'eau collectées sur des moyens d'égouttage 2, notamment des égouttoirs 2, à proximité de la rive du quai.

Le transfert des jacinthes d'eau récoltées est réalisé à l'aide de moyens de transfert 3. Selon un mode de réalisation particulier, lorsque le terrain permet l'installation de rails, les moyens de transfert 3 peuvent consister notamment en des chariots télescopiques rotatifs capables de transférer les jacinthes du bateau vers les égouttoirs 2 de la rive du quai.

Avantageusement, les égouttoirs 2 sont positionnés à proximité de la rive bordant le milieu aquatique.

Selon un mode de réalisation particulier, les égouttoirs 2 consistent en des grilles métalliques inoxydables qui sont surélevées par rapport au sol.

Le caractère inoxydable est essentiel pour éviter une usure prématurée des égouttoirs 2 par l'excédent d'eau et éviter la corrosion et la dégradation par la rouille.

Le fait d'égoutter les jacinthes d'eau récoltées permet une première élimination grossière de l'excédent d'eau afin de faciliter le traitement des jacinthes.

Selon un mode de réalisation particulier de l'invention, on récupère l'excédent d'eau, c'est-à-dire les eaux résiduelles issues de l'égouttage. Puis, on traite lesdites eaux résiduelles par stérilisation UV au sein d'une station d'épuration 4, pour les rejeter par la suite dans le milieu aquatique d'origine des jacinthes d'eau.

Après ce premier égouttage grossier sur les égouttoirs 2, on lave les jacinthes d'eau égouttées avec de l'eau propre. Selon un mode de réalisation particulier, l'eau propre de lavage consiste en de l'eau prélevée à partir du milieu aquatique d'origine des jacinthes d'eau. Ladite eau prélevée est pompée à l'aide d'une station de pompage 5 et filtrée sur des grillages fins d'écartement de diamètre compris entre 6 et 10 mm. Puis, ladite eau est tamisée sur des tamis avec des perforations comprises entre 4 et 6 mm afin de la rendre propre pour l'étape de lavage.

Avantageusement, le lavage est réalisé à l'aide de moyens de lavage 6, notamment dans un conteneur de lavage 6, avec une eau propre.

De manière avantageuse, on répète plusieurs fois l'opération de lavage.

Avantageusement, on lave au moins deux fois la même quantité de matière. Néanmoins, en fonction de la pollution et de la composition du milieu aquatique où les jacinthes ont été prélevées, c'est-à-dire en fonction de la saleté dudit milieu aquatique de vie des jacinthes d'eau, des lavages supplémentaires seront nécessaires. Ces lavages pouvant aller jusqu'à dix lavages successifs.

L'étape de lavage permet d'éliminer les impuretés liées et encore présentes dans le mou de jacinthes d'eau égouttées et de les purifier grossièrement.

Après lavage, on égoutte sur tapis vibrant 7 les jacinthes d'eau lavées pour éliminer en partie le surplus d'eau de lavage et obtenir une matière la moins hydratée possible.

Selon un mode de réalisation particulier de l'invention, les tapis vibrants 7 se présentent sous la forme d'égouttoirs vibrants inclinés à environ 5° et dont la grille filtrante présente des ouvertures à diamètre dégressif allant de 2 à 0,2 mm.

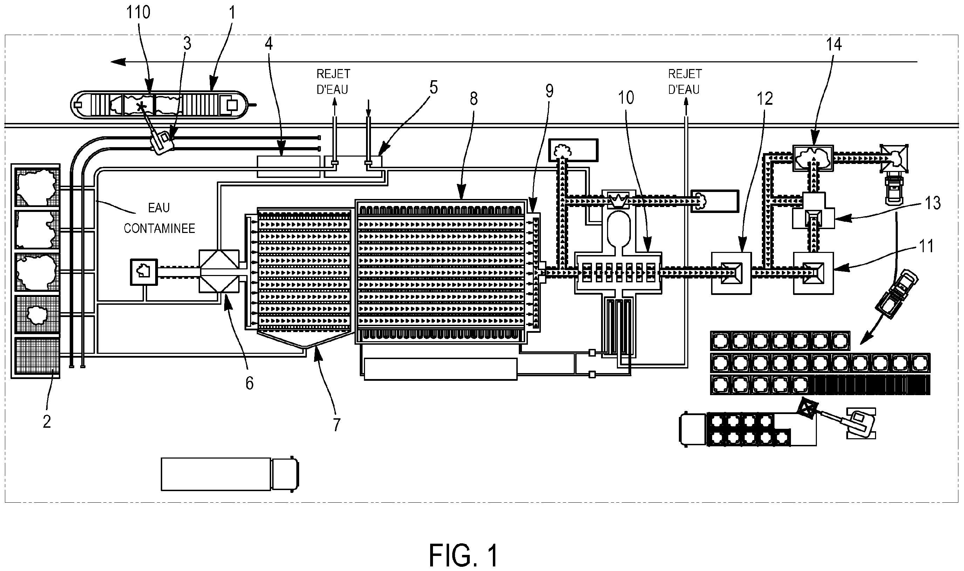

Avantageusement, la matière, c'est-à-dire la jacinthe d'eau lavée, est égouttée sur une série de tapis 7 roulants vibrants en grillage métallique inoxydable tel qu'illustré sur la figure 1.

Selon un mode de réalisation particulier, les résidus d'eau, après passage sur les tapis roulants vibrants 7, sont récupérés pour être stérilisés aux UV et rejetés dans le milieu aquatique.

Selon l'invention, on sèche les jacinthes d'eau à l'aide de moyens de séchage 8, en particulier sur un séchoir 8. On sèche à une température comprise entre 50 et 110°C pendant une durée comprise entre 5h et 16h.

Selon un mode de réalisation spécifique, ledit séchage des jacinthes d'eau est réalisé à une température comprise entre 50 et 70°C pendant une durée comprise entre 14h et 16h.

Selon un autre mode de réalisation privilégié, ledit séchage des jacinthes d'eau est réalisé à une température entre 90 et 110°C pendant une durée comprise entre 5h et 7h.

Dans tous les cas, le séchage doit se faire à une température inférieure à 110°C pour éviter d'abîmer les fibres des jacinthes.

Selon un mode de réalisation particulier, ledit séchoir 8 consiste en un séchoir vertical à air chaud 8 alimenté à l'énergie solaire notamment pour des économies d'énergies nécessaires à son fonctionnement, ou, par tout autre système d'alimentation en énergie.

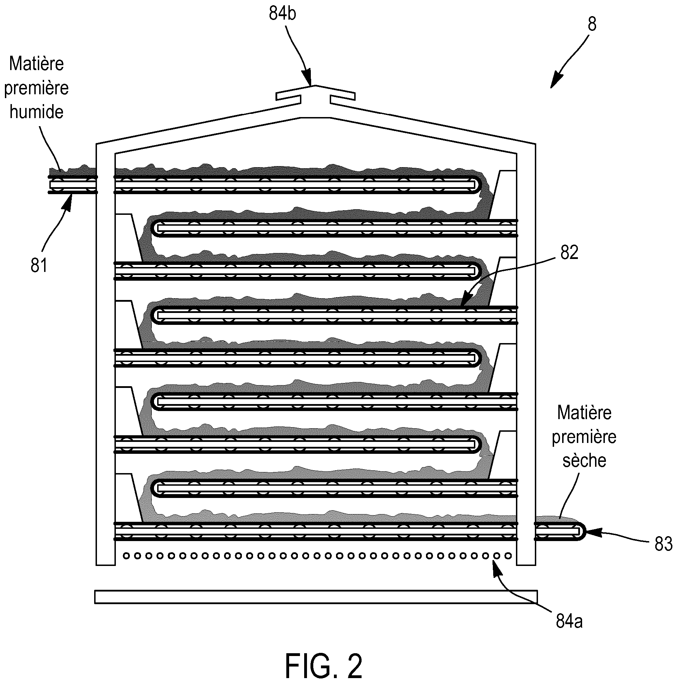

Selon ce mode de réalisation particulier et tel qu'illustré sur la figure 2, ce séchoir vertical à air chaud 8 comporte :
- en partie supérieure une entrée 81, de type conduit, permettant l'incorporation de la matière lavée humide à sécher ;
- sur toute sa hauteur, une série de tapis roulants grillagés 82 inoxydables qui vibrent et permettent le transport progressif, du haut vers le bas, de la matière à sécher, les grilles permettant le passage de l'air ;
- en partie inférieure, une sortie 83, de type conduit, permettant l'évacuation de la matière séchée, c'est-à-dire des jacinthes d'eau séchées, vers l'extérieur du séchoir vertical à air chaud 8.

Il est à noter que les tapis roulants 82 sont vibrants et évitent ainsi un séchage statique de la matière. En effet, un séchage statique est plus lent et moins efficace qu'un séchage dynamique par vibration.

Dans ledit séchoir vertical à air chaud 8, la présence en partie inférieure d'un système d'entrée d'air 84a et en partie supérieure d'une ouverture d'évacuation d'air 84b va permettre de sécher progressivement la matière. La circulation d'air à l'intérieur du séchoir va ainsi faciliter le séchage. Dans ce séchoir 8, la circulation d'air chaud possible sur toute la hauteur, au travers notamment des grilles, va permettre un séchage progressif de la matière. L'air chaud circulant au travers du séchoir 8 a une température comprise entre 50 et 110°C.

En sortie 83 du séchoir 8, on récupère la matière séchée et on concentre les jacinthes d'eau séchées à l'aide de moyens de concentration 9, en particulier dans un concentrateur 9. L'utilisation du concentrateur 9 permet de fluidifier la ligne d'action et de circulation de la matière.

Après passage dans le séchoir 8, on stérilise à la vapeur, à une température comprise entre 80 et 100°C, la matière concentrée, afin de stériliser les jacinthes d'eau séchées et leurs graines. La stérilisation est réalisée à l'aide de moyens de stérilisation à vapeur 10, en particulier dans un stérilisateur à vapeur 10.

Ce procédé de stérilisation et la gamme de température utilisée permettent d'éviter une germination des graines de jacinthes d'eau et leur prolifération. La stérilisation permet d'empêcher ladite germination possible notamment lorsque l'on réalise l'étape de broyage.

Selon le procédé de l'invention, afin de finaliser le traitement des jacinthes d'eau séchées, on broie les jacinthes d'eau et leurs graines stérilisées, afin de pouvoir les valoriser ultérieurement et industriellement.

L'étape de broyage est réalisée à l'aide de moyens de broyage 11, notamment de type broyeur 11, permettant d'obtenir des broyats de matière présentant une granulométrie comprise entre 1 et 3 cm. Cette taille de broyat est nécessaire pour une facilité d'utilisation en industrie, notamment dans le domaine de la plasturgie et les matériaux d'isolation.

Selon un mode de réalisation particulier, afin d'optimiser le broyage et pour obtenir une matière la plus sèche possible, avec un taux d'hydratation sur matière sèche inférieur à 20 % d'HR ou « d'Humidité Relative », il est possible de réaliser après l'étape de stérilisation g) et avant l'étape de broyage h), un séchage par ventilation de la matière stérilisée, c'est-à-dire des jacinthes d'eau stérilisées, par un système d'air chaud pulsé. Ce séchage par ventilation est réalisé dans un séchoir à air pulsé 12.

Le système d'air chaud pulsé permet de déshydrater encore davantage la jacinthe stérilisée, notamment en la mettant en contact avec de l'air chaud à une température comprise entre 50 et 70°C.

Selon un autre mode de réalisation spécifique de l'invention, après l'étape de broyage h), on réalise une micronisation des broyats obtenus afin de permettre leur utilisation et leur valorisation dans le domaine de la plasturgie et des matériaux d'isolation.

La micronisation est réalisée dans un microniseur 13 permettant d'obtenir une granulométrie des broyats inférieure à 1mm.

Ce type de broyats à très faible granulométrie est facilement valorisable.

Selon un autre mode de réalisation de l'invention, les broyats de jacinthes d'eau peuvent être tamisés à l'aide d'un tamiseur 14 pour que leur forme granulométrique soit facile d'utilisation. Les refus du tamiseur 14 peuvent servir de combustible pour alimenter une chaudière à vapeur, permettant ainsi le fonctionnement du stérilisateur à vapeur 10.

La présente invention concerne également une unité de récolte et le traitement des jacinthes d'eau illustré à la figure 1 comprenant dans cet ordre :
- Une barge (1) flottante à proximité de la rive, ladite barge (1) étant équipée de moyens de coupe des jacinthes d'eau, de moyens de collecte et de stockage (110) des jacinthes d'eau découpées, et de moyens de transfert (3) desdites jacinthes d'eau vers la rive, lesdits moyens de transfert (3) étant destinés à transférer lesdites jacinthes d'eau vers des moyens d'égouttage (2) destinés à égoutter les jacinthes d'eau collectées ;
- Des moyens de déplacement des jacinthes d'eau égouttées vers des moyens de lavage (6) connectés à des moyens d'égouttage supplémentaires ;
- Des moyens de séchage (8) en sortie desdits moyens d'égouttage supplémentaires ;
- Des moyens de concentration (9) des jacinthes d'eau séchées en sortie desdits moyens de séchage (8) ;
- Des moyens de stérilisation (10) des jacinthes d'eau concentrées en sortie desdits moyens de concentration (9) ;
- Des moyens de broyage (11) des jacinthes d'eau stérilisées en sortie desdits moyens de stérilisation (10).

Selon un mode de réalisation particulier, l'unité de récolte et de traitement des jacinthes d'eau peut comporter en plus, en sortie du stérilisateur à vapeur 10, un séchoir 12 pour éliminer encore davantage l'eau restante.

Selon un autre mode de réalisation particulier de l'invention, en sortie du broyeur 11, les broyats de jacinthes d'eau déshydratées sont micronisés dans un microniseur 13, puis de manière optionnelle, les broyats micronisés sont tamisés à l'aide d'un tamiseur 14. Ainsi, ce traitement spécifique permet de faciliter leur valorisation industrielle, la « poudre » micronisée et/ou tamisée de jacinthes obtenue étant stable, inerte, sans risque de germination et transportable facilement.

Ainsi, l'unité de récolte et de traitement des jacinthes d'eau permet à la fois de récolter et de traiter les jacinthes d'eau en les transformant en broyats valorisables en industrie. Lesdits broyats obtenus présentent un taux d'hydratation si faible, presque nul, que le risque de germination au stockage a été éliminé. Ainsi, les broyats se conservent durablement et sont inertes, ils peuvent être utilisés en industrie par exemple dans le domaine de la plasturgie ou de la fabrication des panneaux d'isolation.

Le procédé de récolte et de traitement des jacinthes d'eau et son unité de récolte représentent une solution facile à mettre en œuvre pour dépolluer les milieux aquatiques et éliminer les jacinthes d'eau. Le procédé et l'unité de récolte de l'invention permettent également un traitement rapide et efficace des jacinthes d'eau récoltées en vue de pouvoir les valoriser industriellement.

## Revendications

1. Procédé de récolte et de traitement des jacinthes d'eau **caractérisé en ce que** l'on réalise les étapes suivantes :
a) on coupe les jacinthes d'eau présentes dans un milieu aquatique ;
b) on collecte les jacinthes d'eau à bord d'une barge (1) à quai ;
c) on transfère les jacinthes d'eau collectées sur des moyens d'égouttage (2), de type égouttoirs (2), à proximité de la rive du quai ;
d) on lave les jacinthes égouttées avec de l'eau propre ;
e) on égoutte sur tapis vibrants (7) les jacinthes d'eau lavées pour éliminer en partie le surplus d'eau ;
f) on sèche les jacinthes d'eau sur un séchoir (8) à une température comprise entre 50 et 100°C pendant une durée comprise entre 5h et 17h ;
g) on stérilise à la vapeur à une température comprise entre 80 et 100°C, afin de stériliser les jacinthes d'eau et leurs graines ;
h) on broie les jacinthes d'eau et leurs graines stérilisées afin de les valoriser.

2. Procédé de récolte et de traitement des jacinthes d'eau selon la revendication précédente **caractérisé en ce qu'**on récupère les eaux résiduelles issues de l'égouttage de l'étape c), on traite lesdites eaux résiduelles par stérilisation UV au sein d'une station d'épuration (4) pour les rejeter dans le milieu aquatique d'origine des jacinthes d'eau.

3. Procédé de récolte et de traitement des jacinthes d'eau selon la revendication 1 **caractérisé en ce que** l'eau propre de l'étape d) consiste en de l'eau prélevée à partir du milieu aquatique d'origine des jacinthes d'eau, ladite eau prélevée étant pompée à l'aide d'une station de pompage (5) et filtrée au travers d'une grille filtrante comprenant un diamètre d'ouverture compris entre 6 et 10 mm de manière à la rendre propre pour l'étape de lavage.

4. Procédé de récolte et de traitement des jacinthes d'eau selon la revendication 1 dans lequel on réalise l'étape d) en lavant au moins deux fois à l'eau propre les jacinthes égouttées dans des moyens de lavage (6), de type conteneur de lavage (6).

5. Procédé de récolte et de traitement des jacinthes d'eau selon la revendication 1 **caractérisé en ce qu'**après l'étape g) de stérilisation et avant l'étape h) de broyage, on sèche les jacinthes d'eau stérilisées par un système d'air chaud pulsé.

6. Procédé de récolte et de traitement des jacinthes d'eau selon la revendication 1 **caractérisé en ce qu'**après l'étape h) de broyage, on réalise une micronisation des broyats obtenus afin de permettre leur utilisation et leur valorisation dans le domaine de la plasturgie ou des matériaux d'isolation.

7. Procédé de récolte et de traitement des jacinthes d'eau selon la revendication 1 **caractérisé en ce que** l'étape f) de séchage est réalisée à l'aide d'un séchoir (8) vertical à air chaud.

8. Procédé de récolte et de traitement des jacinthes selon la revendication 1 **caractérisé en ce qu'**après l'étape de séchage f) et avant l'étape g) de stérilisation, on concentre les jacinthes d'eau séchées dans des moyens de concentration (9), de type un concentrateur.

9. Procédé de récolte et de traitement des jacinthes d'eau selon la revendication 1 **caractérisé en ce qu'**après l'étape d) de lavage desdites jacinthes d'eau égouttées, on coupe ces dernières en au moins trois ou quatre tronçons.

10. Unité de récolte et de traitement des jacinthes d'eau **caractérisée en ce qu'**elle comprend :
- une barge (1) flottante à proximité de la rive, ladite barge (1) étant équipée de moyens de coupe des jacinthes d'eau, de moyens de collecte et de stockage (110) des jacinthes d'eau découpées, et de moyens de transfert (3) desdites jacinthes d'eau vers la rive ;
- des moyens d'égouttage (2) destinés à égoutter les jacinthes d'eau collectées, lesdits moyens de transfert (3) étant destinés à transférer lesdites jacinthes d'eau vers lesdits moyens d'égouttage (2) ;
- des moyens de lavage (6) ;
- des moyens de déplacement des jacinthes d'eau égouttées vers lesdits moyens de lavage (6) ;
- des moyens d'égouttage supplémentaires ;
- lesdits moyens de lavage (6) étant connectés auxdits moyens d'égouttage supplémentaires ;
- des moyens de séchage (8) en sortie desdits moyens d'égouttage supplémentaires ;
- des moyens de concentration (9) des jacinthes d'eau séchées en sortie desdits moyens de séchage (8) ;
- des moyens de stérilisation (10) des jacinthes d'eau concentrées en sortie desdits moyens de concentration (9) ;
- des moyens de broyage (11) des jacinthes d'eau stérilisées en sortie desdits moyens de stérilisation (10).

## Patentansprüche

1. Verfahren zum Sammeln und Behandeln von Wasserhyazinthen, **dadurch gekennzeichnet, dass** die folgenden Schritte ausgeführt werden:
a) die in einer aquatischen Umgebung vorhandenen Wasserhyazinthen, werden geschnitten;
b) die Wasserhyazinthen werden an Bord eines Lastkahns (1) am Anlegeplatz gesammelt;
c) die gesammelten Wasserhyazinthen werden auf Entwässerungsmittel (2), wie Entwässerungsflächen (2), nahe dem Ufer des Kais verlagert;
d) die entwässerten Hyazinthen werden mit sauberem Wasser gewaschen;
e) die gewaschenen Wasserhyazinthen werden auf Vibrationsmatten (7) entwässert, um das überschüssige Wasser teilweise zu entfernen;
f) die Wasserhyazinthen werden in einem Trockner (8) bei einer Temperatur zwischen 50 und 100°C während einem Zeitraum zwischen 5 und 17 Stunden getrocknet;
g) es wird bei einer Temperatur zwischen 80 und 100°C mit Dampf sterilisiert, um die Wasserhyazinthen und ihre Samen zu sterilisieren;
h) die sterilisierten Wasserhyazinthen und ihre Samen werden zerkleinert, um sie verwenden zu können.

2. Verfahren zum Sammeln und Behandeln von Wasserhyazinthen nach dem vorhergehenden Anspruch, **dadurch gekennzeichnet, dass** das aus der Entwässerung von Schritt c) erhaltene Restwasser gewonnen wird, das besagte Restwasser durch UV-Sterilisation innerhalb einer Kläranlage (4) behandelt wird, um es zurück in die ursprüngliche aquatische Umgebung der Wasserhyazinthen zu leiten.

3. Verfahren zum Sammeln und Behandeln von Wasserhyazinthen nach Anspruch 1, **dadurch gekennzeichnet, dass** das saubere Wasser aus dem Schritt d) aus Wasser besteht, das aus der ursprünglichen aquatischen Umgebung der Waaserhyazinthen entnommen wurde, wobei das besagte entnommene Wasser mittels einer Pumpstation (5) gepumpt und durch ein Filtergitter mit einem Öffnungsdurchmesser zwischen 6 und 10 mm gefiltert wird, sodass es für den Schritt des Waschens gesäubert wird.

4. Verfahren zum Sammeln und Behandeln von Wasserhyazinthen nach Anspruch 1, wobei der Schritt d) durchgeführt wird, indem die entwässerten Hyazinthen mindestens zweimal mit sauberem Wasser in Waschmitteln (6), der Art Waschbehälter (6), gewaschen werden.

5. Verfahren zum Sammeln und Behandeln von Wasserhyazinthen nach Anspruch 1, **dadurch gekennzeichnet, dass** die sterilisierten Wasserhyazinthen nach dem Schritt g) der Sterilisation und vor dem Schritt h) des Zerkleinerns mittels eines pulsierenden Heißluftsystems getrocknet werden.

6. Verfahren zum Sammeln und Behandeln von Wasserhyazinthen nach Anspruch 1, **dadurch gekennzeichnet, dass** nach dem Schritt h) des Zerkleinerns eine Mikronisierung des erhaltenen Mahlguts durchgeführt wird, um dessen Verwendung und Rückgewinnung auf dem Gebiet der Kunststoffverarbeitung oder der Isoliermaterialien zu ermöglichen.

7. Verfahren zum Sammeln und Behandeln von Wasserhyazinthen nach Anspruch 1, **dadurch gekennzeichnet, dass** der Schritt f) des Trocknens mittels eines vertikalen Heißlufttrockners (8) durchgeführt wird.

8. Verfahren zum Sammeln und Behandeln von Hyazinthen nach Anspruch 1, **dadurch gekennzeichnet, dass** die getrockneten Wasserhyazinthen nach dem Schritt f) des Trocknens und vor dem Schritt g) der Sterilisation in Konzentrationsmitteln (9), der Art Konzentratortyp, konzentriert werden.

9. Verfahren zum Sammeln und Behandeln von Wasserhyazinthen nach Anspruch 1, **dadurch gekennzeichnet, dass** nach dem Schritt d) des Waschens der besagten entwässerten Wasserhyazinthen letztere in mindestens drei oder vier Abschnitte geschnitten werden.

10. Einheit zum Sammeln und Verarbeiten von Wasserhyazinthen, **dadurch gekennzeichnet, dass** sie Folgendes umfasst:
- ein Lastkahn (1), der nahe am Ufer schwimmt, wobei der besagte Lastkahn (1) mit Mitteln zum Schneiden der Wasserhyazinthen, Mitteln zum Sammeln und Speichern (110) der geschnittenen Wasserhyazinthen und Mitteln zum Verlagern (3) der besagten Wasserhyazinthen auf den Ufer ausgestattet ist;
- Entwässerungsmittel (2) zum Entwässern der gesammelten Wasserhyazinthen, wobei die besagten Mittel zum Verlagern (3) dazu bestimmt sind, die besagten Wasserhyazinthen auf die besagten Entwässerungsmittel (2) zu verlagern;
- Waschmittel (6);
- Mittel zum Bewegen der entwässerten Wasserhyazinthen zu den besagten Waschmitteln (6);
- zusätzliche Entwässerungsmittel;
- wobei die besagten Waschmittel (6) mit den besagten zusätzlichen Entwässerungsmitteln verbunden sind;
- Trocknungsmittel (8) am Auslass der besagten zusätzlichen Entwässerungsmittel;
- Mittel zum Konzentrieren (9) der getrockneten Wasserhyazinthen am Auslass der besagten Trocknungsmittel (8);
- Mittel (10) zum Sterilisieren der konzentrierten Wasserhyazinthen am Auslass der besagten Konzentrationsmittel (9);
- Mittel zum Zerkleinern (11) der sterilisierten Wasserhyazinthen am Auslass der besagten Sterilisationsmittel (10).

## Claims

1. A method for collecting and treating water hyacinths, wherein the following steps are performed:
a) the water hyacinths present in an aquatic environment are cut;
b) the water hyacinths are collected on board a barge (1) alongside the quay;
c) the collected water hyacinths are transferred onto draining means (2), such as drain-boards (2), near the quay shore;
d) the drained hyacinths are washed with clean water;
e) the washed water hyacinths are drained on vibrating mats (7) in order to partially remove the excess water;
f) the water hyacinths are dried on a dryer (8) at a temperature between 50 and 100°C for a period between 5h and 17h;
g) a steam sterilization at a temperature between 80 and 100°C is performed, in order to sterilize the water hyacinths and their seeds;
h) the water hyacinths and their sterilized seeds are ground in order to recover them for use.

2. The method for collecting and treating water hyacinths according to the preceding claim, wherein the residual water resulting from the draining of step c) is recovered, said residual water is treated by UV sterilization in a treatment plant (4) in order to discharge it into the original aquatic environment of the water hyacinths.

3. The method for collecting and treating water hyacinths according to claim 1, wherein the clean water from step d) consists of water withdrawn from the original aquatic environment of the water hyacinths, said withdrawn water being pumped using a pumping station (5) and filtered through a filtering grid comprising an opening diameter between 6 and 10 mm so as to make it clean for the washing step.

4. The method for collecting and treating water hyacinths according to claim 1, wherein step d) is carried out by washing the drained hyacinths at least twice with clean water in washing means (6), such as a washing container (6).

5. The method for collecting and treating water hyacinths according to claim 1, wherein, after the step g) of sterilization and before the step h) of grinding, the sterilized water hyacinths are dried by a forced hot air system.

6. The method for collecting and treating water hyacinths according to claim 1, wherein, after the step h) of grinding, a micronization of the obtained ground material is carried out in order to permit their use and recovery in the field of plastics processing or insulation materials.

7. The method for collecting and treating water hyacinths according to claim 1, wherein the step f) of drying is carried out using a vertical hot air dryer (8).

8. The method for collecting and treating hyacinths according to claim 1, wherein, after the step f) of drying and before the step g) of sterilization, the dried water hyacinths are concentrated in concentrating means (9), such as a concentrator.

9. The method for collecting and treating water hyacinths according to claim 1, wherein, after the step d) of washing said drained water hyacinths, the latter are cut into at least three or four sections.

10. A unit for collecting and processing water hyacinths, wherein it comprises:
- a barge (1) floating close to the shore, said barge (1) being equipped with means for cutting the water hyacinths, means for collecting and storing (110) the cut water hyacinths, and means for transferring (3) said water hyacinths to the shore;
- draining means (2) for draining the collected water hyacinths, said transferring means (3) being intended to transfer said water hyacinths to said draining means (2);
- washing means (6);
- means for moving the drained water hyacinths to said washing means (6);
- additional drainage means;
- said washing means (6) being connected to said additional draining means;
- drying means (8) at the outlet of said additional draining means;
- means for concentrating (9) the dried water hyacinths at the outlet of said drying means (8);
- means (10) for sterilizing the concentrated water hyacinths at the outlet of said concentrating means (9);
- means for grinding (11) the sterilized water hyacinths at the outlet of said sterilization means (10).
